# EUROPEAN PATENT APPLICATION

(11) **EP 2 731 167 A1**
(43) Date of publication of application: **14.05.2014**
(21) Application number: 13191952.4
(22) Date of filing: 07.11.2013
(51) Int. Cl.: H01M 2/10, A61B 5/024, G01C 22/00, H05K 5/00

(54) **Electronic device**

(30) Priority: 07.11.2012 JP 2012245671
(71) Applicant: Seiko Instruments Inc., Chiba-shi, Chiba (JP)
(72) Inventor: Nakamura, Hisao, Chiba-shi,, Chiba (JP); Kato, Teruo, Chiba-shi, Chiba (JP); Koshoji, Hideaki, Chiba-shi, Chiba (JP); Terasawa, Dai, Chiba-shi, Chiba (JP); Okuda, Hideki, Chiba-shi, Chiba (JP)
(74) Representative: Miller Sturt Kenyon

(57) **Abstract**

An electronic device in which a battery can be positioned in a diameter direction, the battery can be brought into contact with a positive terminal member 50 stably and sufficiently, and a portion housing the battery and a case body can be molded individually from optimal materials is provided. The electronic device 1 includes a battery frame 30 in which a battery is housed, a holding portion 40 removably holding the battery frame on a bottom wall portion 10a such that a battery insertion opening 16 communicates with the interior of the battery frame, and a battery lid 70 removably fixed to the bottom wall portion to cover the battery insertion opening, wherein the battery frame includes a circumferential wall portion 31 surrounding the battery outside in a diameter direction, and a positive terminal holding portion 33 fixing the positive terminal member such that a battery side contact portion 52 of the positive terminal member is disposed inside the circumferential wall portion, and a portion of an inner circumferential face of the circumferential wall portion serves as a battery guide face positioning the battery in the diameter direction and bringing the battery into contact with the battery side contact portion.

## Description

### BACKGROUND OF THE INVENTION

### FIELD OF THE INVENTION

The present invention relates to an electronic device using a battery.

### BACKGROUND ART

A number of electronic devices using button batteries (including ones called coin batteries) have conventionally been known. Examples of those devices include pedometers described in JP-U-3162080 (Patent Literature 1) and JP-A-2009-175940 (Patent Literature 2).

In general, when such a button battery is used, the button battery is often put in a lower case of a case body. Thus, a typically employed structure includes a battery housing portion formed in the lower case and a battery lid attached to the lower case for fixing the button battery housed in the battery housing portion.

The battery housing portion is often formed of a guide side wall for determining the position of the button battery in a diameter direction (plane position) (guide wall in contact with the outer circumferential face of the button battery) and a partition referred to as a battery stage for determining the position of the button battery in a thickness direction. The battery stage is provided for bringing a negative electrode face of the button battery into contact with a negative terminal member attached to the lower case at a proper contact pressure.

In the pedometer described in Patent Literature 1, a lower case has a battery housing portion including a guide side wall formed thereon and has a battery lid attached thereto. In the pedometer, the battery housing portion does not include a battery stage and includes only the guide side wall.

A positive terminal member and a negative terminal member electrically conducting to the button battery are fixed, for example by soldering to a circuit board attached to the lower case, and are in contact with the button battery. This ensures the electrical conduction path between the button battery and the circuit board.

In the pedometer described in Patent Literature 2, a lower case has a battery housing portion formed thereon and a battery lid of a slide type attached thereto. The battery housing portion includes a guide side wall and a battery stage.

A circuit board is attached across the battery stage from the button battery. A negative terminal member electrically conducting to the button battery is fixed to the battery stage and a positive terminal member is fixed to the battery lid.

The negative terminal member is in contact with a circuit pattern on the circuit board and is in contact with the button battery through an opening portion formed in part of the battery stage. The positive terminal member is in contact with the button battery and electrically conducts to the circuit pattern on the circuit board through each of connecting members such as a connecting pin and an electrically conductive spring attached to the lower case. This ensures the electrical conduction path between the button battery and the circuit board.

In the electronic device using the conventional button battery such as the pedometers described in Patent Literatures 1 and 2, the battery housing portion is formed in the case body such as the lower case. For example, in the molding of the case body, the simultaneous molding of the battery housing portion is required. The battery housing portion including the guide side wall and the battery stage, however, has a complicated shape, and it is difficult to precisely mold the battery housing portion simultaneously with the formation of the outer shape of the case body.

In particular, the battery lid is also fixed to the case body, so that the constituent members necessary for fixing the battery lid need to be molded simultaneously. This makes it more difficult to precisely mold the battery housing portion.

By way of example, for fixing the battery lid with a structure of a bayonet type, it is necessary to form a bayonet protrusion of overhang shape near an opening portion in the battery housing portion for engaging with a bayonet hook on the battery lid and to form the guide wall and the battery stage. The precise formation of all these components is difficult, and the battery housing portions after molding easily vary in shape.

This may lead to variations in thickness of the guide side wall of the battery housing portion, and the position of the housed button battery may not be controlled but be shifted in the diameter direction. This reduces resistance to vibrations and shocks to easily degrade the quality. In addition, the movement of the button battery in the diameter direction may inhibit the sufficient contact with the positive terminal member to reduce the operation reliability.

Since the battery housing portion is formed on the case body, the battery housing portion needs to be molded from a resin material which satisfies the requirements of the case body in the electronic device including the strength and various quality characteristics. This impairs the flexibility in selecting materials such as selection of a resin material easy to mold, and there is a need for improvement.

### SUMMARY OF THE INVENTION

It is an aspect of the present application to provide an electronic device in which a battery can be stably positioned by reliably controlling the position thereof in a diameter direction, the battery can be brought into contact with a positive terminal member stably and sufficiently, and a portion housing the battery and a case body can be molded individually from optimal materials.

The present application provides the following.
(1) According to the present application, an electronic device including a case body which accommodates a battery includes a battery frame housed in the case body such that the battery frame is mounted on a bottom wall portion of the case body, the battery is housed in the battery frame, a holding portion provided on the bottom wall portion and removably holding the battery frame on the bottom wall portion such that a battery insertion opening formed in the bottom wall portion communicates with the interior of the battery frame, a circuit board housed in the case body and conducting to the battery through a positive terminal member and a negative terminal member, and a battery lid removably fixed to the bottom wall portion to cover the battery insertion opening, wherein the battery frame includes a circumferential wall portion surrounding the battery housed therein outside in a diameter direction, and a positive terminal holding portion fixing the positive terminal member such that a battery side contact portion of the positive terminal member is disposed inside the circumferential wall portion, and a portion of an inner circumferential face of the circumferential wall portion serves as a battery guide face abutting on an outer circumferential face of the battery to position the battery in the diameter direction and bringing the battery into contact with the battery side contact portion of the positive terminal member.

The electronic device according to the present application includes the battery frame independently of the case body, so the case body and the battery frame can be molded individually unlike the conventional structure including the case body having the integral battery housing portion.

Consequently, the battery frame can be easily molded by using a mold dedicated to the battery frame, and the battery frame can be formed precisely while suppressing variations in shape after the molding. In addition, since the moldability can be given a higher priority in the material selection, the molding can be performed more easily and precisely. Since the case body can also be provided by using a mold dedicated to the case body, the molding can be facilitated, and variations in shape after the molding can be suppressed. The formation of the battery housing portion is not required, so that the case body can be simplified in shape.

Since the battery frame can be precisely formed as described above, the battery can be housed in the battery frame through the battery insertion opening with the battery guide face reliably abutting on the outer circumferential face of the battery to achieve the stable positioning of the battery by the reliable control of the position in the diameter direction. In addition to the stable position of the battery in the diameter direction, the fixing of the positive terminal member to the battery frame through the positive terminal holding portion can provide the stable and sufficient contact between the battery and the battery side contact portion of the positive terminal member. The resulting electronic device can have high quality with high resistance to vibrations, shocks and the like, and improved operation reliability. The eliminated need to solder the positive terminal member, for example to the circuit board, can easily simplify the assembly process.

(2) In the electronic device according to the present application, the battery frame preferably includes a top wall portion connected to the circumferential wall portion and placed between the circuit board and the battery to abut on the battery, and a negative terminal holding portion fixing the negative terminal member onto the top wall portion with a battery side contact portion of the negative terminal member contacting the battery.

In this case, since the battery frame has the top wall portion (battery stage), certain spacing can be provided between the circuit board and the battery. Even if a shock is given during the insertion or drop of the battery, the negative terminal member can be prevented from being crushed or deformed. The resulting electronic device can have higher resistance to vibrations, shocks and the like, and improved operation reliability in which the stable and sufficient contact can be made between the battery and the battery side contact portion of the negative terminal member.

In addition, since it is not necessary to solder the negative terminal member, for example to the circuit board, and the positive terminal member and the negative terminal member can be integrated with the battery frame, the assembly process can be simplified more easily.

(3) In the electronic device according to the present application, the holding portion preferably includes an engagement portion engaging with the battery frame and defining the orientation of the battery frame in a circumferential direction through the engagement.

In this case, since the holding portion can be used to fit the battery frame onto the bottom wall portion by defining the orientation in the circumferential direction, the positive terminal member and the negative terminal member can be positioned at the preset positions on the circuit board. This can reliably provide the conduction path between the battery and the circuit board through the positive terminal member and the negative terminal member.

(4) In the electronic device according to the present application, the battery insertion opening is preferably provided with bayonet protrusions protruding inward in a diameter direction and disposed at intervals in a circumferential direction, and the battery lid is preferably provided with a bayonet hook having a bayonet engagement groove engaging with the bayonet protrusion when the battery lid is rotated relatively to the case body.

In this case, the bayoneting enables the mounting of the battery lid with a single motion and easily reduces the size of the case body as compared with sliding. In particular, since the case body can be molded without consideration of the battery frame, the bayonet protrusion can be formed precisely. This allows the mounting of the battery lid with reduced rattles, and the battery can be easily housed inside the battery frame stably.

(5) In the electronic device according to the present application, a pair of electrically conductive belts connected to the circuit board and contactable with a living body surface are preferably attached to the case body, and the circuit board is preferably provided with a detection circuit portion detecting living body information based on a potential difference produced between the pair of electrically conductive belts.

In this case, the electronic device can be preferably used for a pedometer or a heart beat measuring apparatus, by way of example.

According to the present application, the battery frame housing the battery and the case body can be molded individually from optimal materials to facilitate the manufacture and to perform the easy and precise formation of them. The precise formation of the battery frame allows the stable positioning of the battery by reliably controlling the position thereof in the diameter direction and enables the battery to be brought into contact with the positive terminal member stably and sufficiently. The resulting electronic device can have high quality with improved operation reliability.

### BRIEF DESCRIPTION OF THE DRAWINGS

Embodiments of the present invention will now be described by way of further example only and with reference to the accompanying drawings, in which:
Fig. 1 is a diagram showing an embodiment according to the present invention and is a front view showing the outer appearance of a heart beat measuring apparatus.
Fig. 2 is a section view taken along a line A-A shown in Fig. 1.
Fig. 3 is a rear view showing the outer appearance of the heart beat measuring apparatus shown in Fig. 1.
Fig. 4 is an exploded perspective view of the heart beat measuring apparatus shown in Fig. 1 in which an antenna portion and an upper case are removed.
Fig. 5 is a perspective view of a battery frame shown in Fig. 4 with which a positive terminal member and a negative terminal member are combined.

### DETAILED DESCRIPTION OF THE INVENTION

An embodiment according to the present invention will hereinafter be described with reference to the drawings.

The present embodiment is described by using a heart beat measuring apparatus for measuring heart beat information serving as living body information as an example of the electronic device.

### <Configuration of heart beat measuring apparatus>

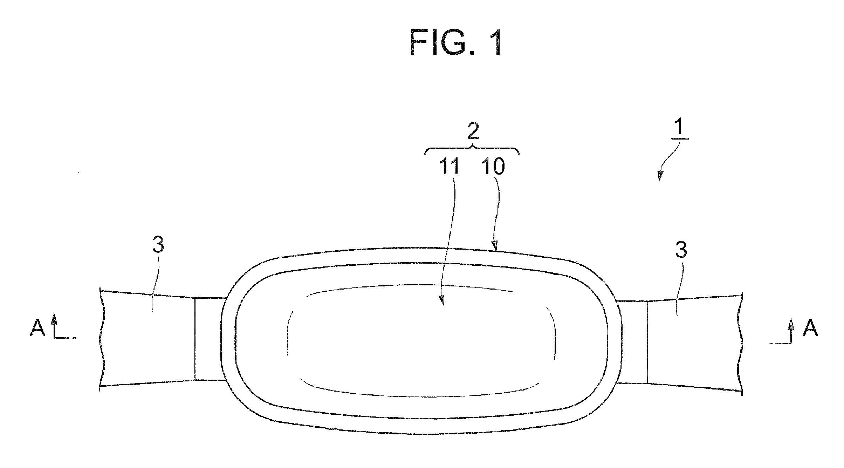

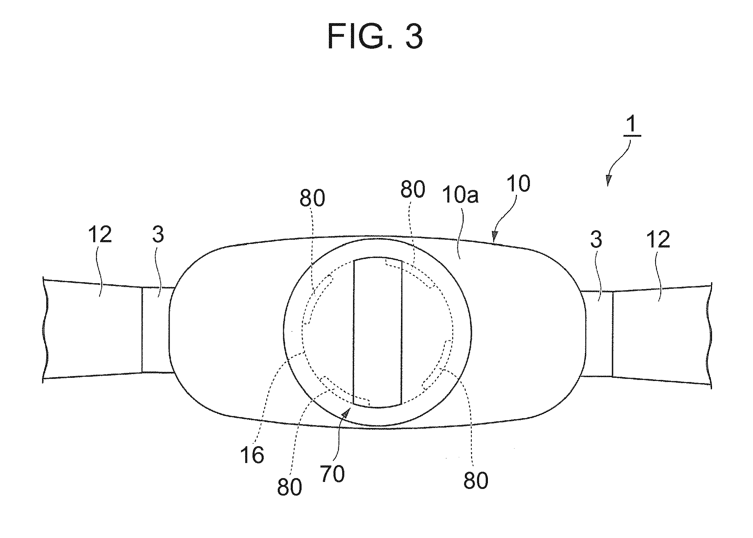

As shown in Fig. 1 to Fig. 3, a heart beat measuring apparatus 1 according to the present embodiment is mounted on the living body surface of a user, not shown, for example the breast, detects an electrocardiographic signal produced with the heart beats, and outputs the detected electrocardiographic signal to the outside, for example through radio communication.

The heart beat measuring apparatus 1 includes a case body 2 elongated in the circumferential direction of the breast of the user, and a pair of fixing belts 3 coupled to each end of the case body 2 in the longitudinal direction for attaching the case body 2 to the breast, and is a portable electronic device which operates on the power from a button battery (battery) 4.

In the following description of the configuration of the heart beat measuring apparatus 1, upper and lower directions are defined such that the lower direction corresponds to the side of the apparatus in contact with the breast of the user and the upper direction corresponds to the opposite side away from the user. The button battery 4 includes one called the coin battery and refers to a battery of a so-called disk type.

The case body 2 includes a lower case 10 placed closer to the breast and an upper case 11 combined with the lower case 10. An internal space K is formed between the lower case 10 and the upper case 11. The button battery 4, later described, a circuit board 25, an antenna portion 27 and the like are put in the internal space K.

A rubber belt (electrically conductive belt) 12 having electrical conductivity is provided integrally on the lower face of each of the pair of fixing belts 3. The pair of rubber belts 12 are in contact with the breast of the user to serve as an electrode portion which detects the potential difference produced between them and outputs the potential difference as an electrocardiographic signal. The pair of rubber belts 12 have connecting portions 12a at the ends thereof closer to the case body 2 and are connected to the circuit board 25 through the circuit connecting portions 12a.

The rubber belt 12 is made of conductive elastomer of strip shape, for example. Examples of the conductive elastomer include electrically conductive silicone rubber mixed with carbon black, electrically conductive rubber mixed with carbon black, and electrically conductive polyurethane rubber mixed with carbon black.

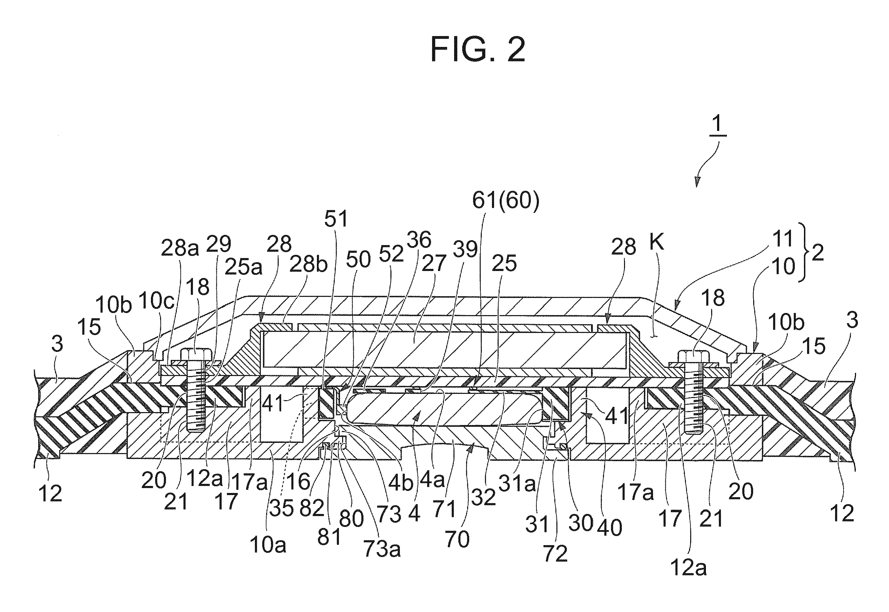

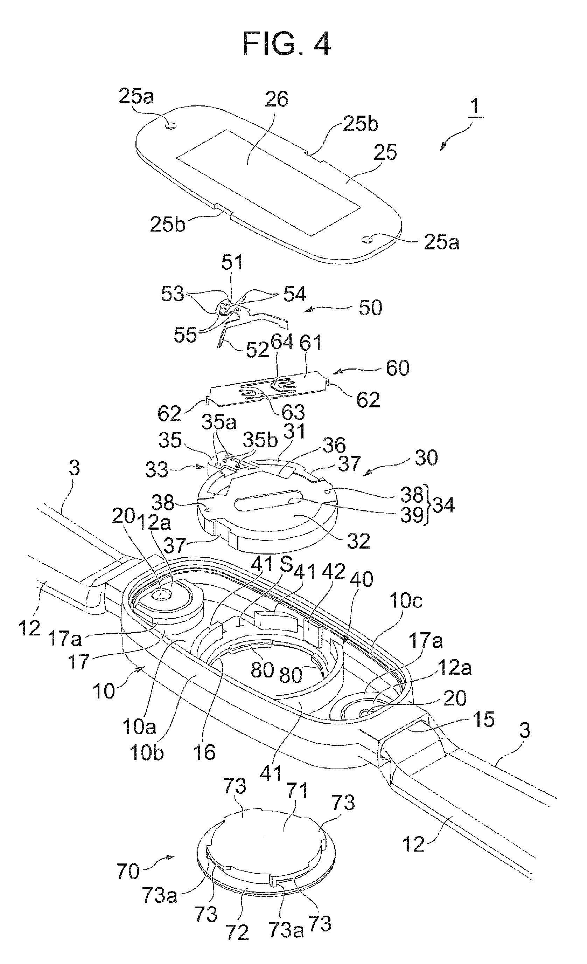

As shown in Fig. 2 and Fig. 4, the lower case 10 is formed in a bottomed cylindrical shape having a bottom wall portion 10a and a frame shape outer wall portion 10b and is formed in a generally oval shape in planar view such that its longer side extends in the circumferential direction of the breast of the user and its shorter side extends in the direction orthogonal to the circumferential direction and the thickness direction.

The outer wall portion 10b has insertion holes 15 at both ends of the lower case 10 in the longitudinal direction for inserting the circuit connecting portions 12a of the pair of rubber belts 12. The bottom wall portion 10a has a battery insertion opening 16 of circular shape in planar view at the center for inserting or removing the button battery 4, and has mount stages 17 at both ends of the lower case 10 in the longitudinal direction for placing the circuit connecting portions 12a inserted into the case body 12 through the insertion holes 15.

The circuit connecting portion 12a has an insertion hole 20 formed therein for allowing the insertion of a fixing screw 18. The mount stage 17 has a female screw portion 21 formed therein at the position associated with the insertion hole 20. The fixing screws 18 are inserted into the insertion holes 20 and are engaged into the female screw portions 21 to fasten and fix the pair of rubber belts 12 to the lower case 10 together with the circuit board 25 and an antenna holding portion 28, later described.

The mount stage 17 has a stopper wall 17a of the shape of the letter C in planar view formed thereon along the end of the circuit connecting portion 12a of the mounted rubber belt 12 to position the circuit connecting portion 12a. The upper end of the stopper wall 17a abuts on the lower face of the circuit board 25 and is responsible for supporting the circuit board 25.

The fixing belt 3 is made of a rubber material such as urethane having no electrical conductivity and is molded integrally with the rubber belt 12. The fixing belt 3 is bonded to each end of the lower case 10 to cover the portion of the rubber belt 12 that is exposed outside the lower case 10.

The lower face of the rubber belt 12 is, however, exposed outside and can be in contact with the breast.

The circuit board 25 has various electronic parts and the like, not shown, necessary for heart beat measurements mounted thereon, and has through holes 25a at both ends for inserting the fixing screws 18. The circuit board 25 has an electrode connecting pattern, not shown, at the position on the lower face associated with the circuit connecting portion 12a of the rubber belt 12. This allows the circuit board 25 to electrically conduct to the circuit connecting portion 12a when the circuit board 25 is fastened and fixed to the lower case 10 by the fixing screw 18.

The circuit board 25 has, on the lower face, a positive pattern and a negative pattern, not shown, electrically conducting to the button battery 4 through a positive terminal member 50 and a negative terminal member 60, later described.

The circuit board 25 has, on the upper face, a detection circuit portion 26 electrically conducting to the electrode connecting pattern and measuring the heart beats based on the electrocardiographic signal detected through the rubber belt 12. The detection circuit portion 26 measures the heart beats as described above and then outputs the information about the heart beats as the electrocardiographic signal to the antenna portion 27.

The antenna portion 27 is mounted on the upper face of the circuit board 25 and is pressed and held onto the circuit board 25 by the antenna holding portion 28. The antenna portion 27 can wirelessly transmit the electrocardiographic signal to the outside, for example a wristwatch or the like worn by the user.

The antenna holding portion 28 is formed in a frame shape surrounding the antennal portion 27 outside in the diameter direction, abuts on the upper face of the circuit board 25, and includes a lower flange portion 28a having a through hole 29 for inserting the fixing screw 18 and an upper flange portion 28b abutting on the upper face of the antennal portion 27 to press the antennal portion 27 toward the circuit board 25.

The antenna holding portion 28 is fastened and fixed to the lower case 10 by the fixing screw 18 together with the circuit board 25 to hold the antennal portion 27 onto the circuit board 25.

As shown in Fig. 1 and Fig. 2, the upper case 11 is a resin cover or a glass cover combined with the lower case 10 to cover the lower case 10 from above, and is fixed through welding or fitting by using a stepped portion 10c formed at the upper end of the outer wall portion 10b of the lower case 10b.

The upper case 11 is formed such that its outer surface smoothly connects to the fixing belt 3 without any height differences and contributes to improving the appearance.

As shown in Fig. 2 and Fig. 4, the heart beat measuring apparatus 1 of the present embodiment includes a battery frame 30 capable of housing the button battery 4 therein.

The battery frame 30 is placed on the bottom wall portion 10a and housed in the case body 2. More specifically, the battery frame 30 is housed in the case body 2 so as to be placed between the bottom wall portion 10a and the circuit board 25 and is removably held by a holding portion 40 provided on the bottom wall portion 10a.

Next, the battery frame 30 is described in detail.

The battery frame 30 is formed in a topped cylindrical shape having a circumferential wall portion 31 of annular shape surrounding the housed button battery 4 outside in the diameter direction, and a top wall portion (battery stage) 32 connected to the circumferential wall portion 31 and placed between the circuit board 25 and the button battery 4 to abut on a negative electrode face 4a of the button battery 4. Thus, the button battery 4 can be housed into the battery frame 30 from the direction of the bottom wall portion 10a.

The circumferential wall portion 31 has a positive terminal holding portion 33 formed thereon for fixing the positive terminal member 50, and the top wall portion 32 has a negative terminal holding portion 34 formed thereon for fixing the negative terminal member 60.

Next, the positive terminal member 50 and the negative terminal member 60 are described.

The positive terminal member 50 is a press-molded article of metal thin plate, for example, and includes a terminal body 51 extending in one direction and in parallel with the circuit board 25, a battery side contact portion 52 bent generally perpendicular toward the bottom wall portion 10a from one end of the terminal body 51 in the longitudinal direction and extending in strip form along the outer circumferential face (positive electrode face) 4b of the button battery 4, a pair of fixing hook portions 53 bent generally perpendicular toward the bottom wall portion 10a from the other end of the terminal body 51 in the longitudinal direction, and a pair of board side contact portions 54 curved toward the circuit board 25 from the intermediate portion of the terminal body 51 in the longitudinal direction.

The battery side contact portion 52 is formed as a leaf spring urged toward the outer circumferential face of the button battery 4, and the board side contact portion 54 is formed as a leaf spring urged toward the circuit board 25. The terminal body 51 has two fitting holes 55 formed therein at an interval in the longitudinal direction of the case body 2.

The negative terminal member 60, similar to the positive terminal member 50, is a press-molded article of metal thin plate, for example, and includes a terminal body 61 extending in one direction, a pair of fixing hook portions 62 bent generally perpendicular toward the bottom wall portion 10a from both ends of the terminal body 61 in the longitudinal direction, a battery side contact portion 63 and a board side contact portion 64 bent so as to curve toward the bottom wall portion 10a and the circuit board 25, respectively, from the intermediate portion of the terminal body 61 in the longitudinal direction.

The battery side contact portion 63 is formed as a leaf spring urged toward the negative electrode face 4a of the button battery 4, and the board side contact portion 64 is formed as a leaf spring urged toward the circuit board 25.

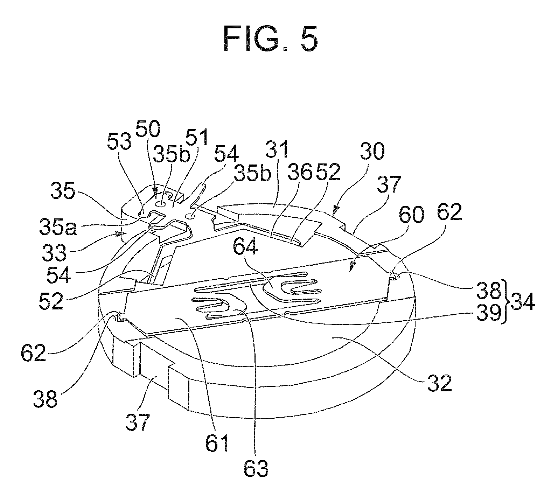

The positive terminal member 50 and the negative terminal member 60 configured in this manner are fixed to the battery frame 30 through the positive terminal holding portion 33 and the negative terminal holding portion 34, respectively, as shown in Fig. 5.

Next, this configuration is described in detail.

As shown in Fig. 4 and Fig. 5, the circumferential wall portion 31 of the battery frame 30 has a stage 35 integrally molded to protrude outward in the diameter direction for mounting the terminal body 51 of the positive terminal portion 50. The upper end face of the stage 35 is formed to be lower than the upper end face of the circumferential wall portion 31 by the thickness of the terminal body 51. This allows the terminal body 51 mounted on the stage 35 to be flush with the upper end face of the circumferential wall portion 31 of the terminal body 51. The upper end face of the circumferential wall portion 31 abuts on the lower face of the circuit board 25 (see Fig. 2) and is also responsible for supporting the circuit board 25.

The stage 35 has a pair of fixing holes 35a into which the pair of fixing hook portions 53 of the positive terminal member 50 are fitted and has a fitting protrusion 35b formed to protrude which is inserted into the fitting hole 55. This allows the terminal body 51 to be mounted on the stage 35 with the pair of fixing hook portions 53 fitted and fixed into the pair of fixing holes 35a and the fitting protrusion 35b fitted and fixed into the fitting hole 55, thereby reliably securing the positive terminal member 50 to the battery frame 30.

The top wall portion 32 has a first terminal opening portion 36 formed therein along the circumferential wall portion 31 for avoiding interference with the battery side contact portion 52 of the positive terminal member 50. When the positive terminal member 50 is fixed to the battery frame 30, the battery side contact portion 52 is placed inside the circumferential wall portion 31.

The stage 35, the pair of fixing holes 35a, the fitting protrusion 35b, and the first terminal opening portion 36 described above serve as the positive terminal holding portion 33 for fixing the positive terminal member 50 with the battery side contact portion 52 placed inside the circumferential wall portion 31.

The board side contact portion 54 of the positive terminal member 50 contacts, by its spring force, the positive pattern formed on the circuit board 25 at a proper pressure and conducts thereto, and the battery side contact portion 52 contacts, by its spring force, the outer circumferential face 4b of the button battery 4 at a proper pressure and conducts thereto.

As shown in Fig. 2, the portion of the inner circumferential face of the circumferential wall portion 31 serves as a battery guide face 31a abutting on the outer circumferential face 4b of the button battery 4 to position the button battery 4 in the diameter direction and reliably bringing the button battery 4 into contact with the battery side contact portion 52 of the positive terminal member 50.

The outer circumferential face of the circumferential wall portion 31 has recess portions 37 dented inward in the diameter direction formed opposite to each other in the diameter direction.

As shown in Fig. 4 and Fig. 5, the negative terminal member 60 is mounted on the upper face of the top wall portion 32. The upper face of the top wall portion 32 is formed to be lower than the upper end face of the circumferential wall portion 31 by the thickness of the terminal body 61. The portion of the upper end face of the circumferential wall portion 31 where the negative terminal member 60 is mounted is also formed to be lower by the thickness of the terminal body 61. This allows the terminal body 61 mounted on the upper face of the top wall portion 32 to be flush with the upper end face of the circumferential wall portion 31.

The portion of the upper end face of the circumferential wall portion 31 formed to be lower by the thickness described above has a pair of fixing holes 38 formed therein into which the pair of fixing hook portions 62 of the negative terminal member 60 are fitted. The top wall portion 32 also has a second terminal opening portion 39 formed therein for avoiding interference with the battery side contact portion 63.

With this structure, the terminal body 61 can be mounted on the upper face of the top wall portion 32 with the pair of fixing hook portions 62 fitted and fixed in the pair of fixing holes 38 to reliably fix the negative terminal member 60 to the battery frame 30. The battery side contact portion 63 protrudes inside the battery frame 30 through the second terminal opening portion 39 to be contactable with the negative electrode face 4a of the button battery 4.

The pair of fixing holes 38 and the second terminal opening portion 39 serve as the negative terminal holding portion 34 for fixing the negative terminal member 60 to the upper face of the top wall portion 32 with the battery side contact portion 63 contacting the button battery 4.

The board side contact portion 64 of the negative terminal member 60 contacts, by its spring force, the negative pattern formed on the circuit board 25 at a proper pressure and conducts thereto, and the battery side contact portion 63 contacts, by its spring force, the negative electrode face 4a of the button battery 4 at a proper pressure and conducts thereto.

As shown in Fig. 2, the battery frame 30 configured in this manner is removably held by the holding portion 40 provided on the bottom wall portion 10a. Next, the holding portion 40 is described.

As shown in Fig. 2 and Fig. 4, the holding portion 40 includes a plurality of guide walls 41 of arc shape standing on the bottom wall portion 10a to be positioned outside the circumferential wall portion 31 of the battery frame 30 in the diameter direction to abut on the outer circumferential face of the circumferential wall portion 31 and placed at spacings in the circumferential direction of the circumferential wall portion 31. The battery frame 30 is inserted inside the plurality of guide walls 41 to be removably held as positioned in the diameter direction.

The height of the guide wall 41 is equal to the thickness of the circumferential wall portion 31 of the battery frame 30, and the upper end face of the guide wall 41 abuts on the lower face of the circuit board 25. Thus, the guide wall 41 is also responsible for supporting the circuit board 25.

One of the spacings between the guide walls 41 is defined as a spacing S (see Fig. 4) for accommodating the stage 35 protruding outward in the diameter direction from the circumferential wall portion 31. In the spacings between the remaining guide walls 41, a pair of engagement walls (engagement portions) 42 stand to engage with the recess portion 37 formed in the outer circumferential face of the circumferential wall portion 31. The battery frame 30 is inserted such that the engagement wall 42 is engaged with the recessed portion 37 and the stage 35 is accommodated in the spacing S, so that the circumferential orientation of the battery frame 30 can be determined.

The engagement wall 42 protrudes to the circuit board 25 and is also responsible for positioning the circuit board 25 through engagement with an engagement recess portion 25b formed in the circuit board 25.

As shown in Fig. 2 and Fig. 4, a battery lid 70 for closing the battery insertion opening 16 is removably fixed to the bottom wall portion 10a of the lower case 10 with bayoneting. Next, this configuration is described.

The battery insertion opening 16 has a plurality of bayonet protrusions 80 formed thereon to protrude inward in the diameter direction and placed at intervals in the circumferential direction. In the example shown, four bayonet protrusions 80 are formed. Each of the bayonet protrusions 80 is formed to extend in the circumferential direction.

As shown in Fig. 2, the bottom wall portion 10a has an annular stepped portion 81 surrounding the battery insertion opening 16 outside in the diameter direction and communicating with the battery insertion opening 16. An O-ring 82 is attached to the stepped portion 81.

As shown in Fig. 2 and Fig. 4, the battery lid 70 includes a lid body 71 of circular shape in planar view placed inside the battery insertion opening 16 and a flange portion 72 of annular shape protruding outward in the diameter direction from the lid body 71 and abutting on the stepped portion 81.

The lid body 71 has bayonet hooks 73 formed thereon to protrude outward in the diameter direction and each having a bayonet engagement groove 73a engaging with the bayonet protrusion 80 when the battery lid 70 is rotated relatively to the lower case 10. The four bayonet hooks 73 are formed at intervals in the circumferential direction in association with the bayonet protrusions 80.

When the engagement of the bayonet protrusion 80 with the bayonet engagement groove 73a fixes the battery lid 70, the flange portion 72 abuts on the stepped portion 81 through the O-ring 82 and the lid body 71 abuts on the button battery 4 to hold and fix the button battery 4 between the lid body 71 and the top wall portion 32 of the battery frame 30.

### <Assembly of heart beat measuring apparatus>

Next, description is made of how to assemble the heart beat measuring apparatus 1 configured as above.

First, as shown in Fig. 4, the circuit connecting portions 12a of the pair of rubber belts 12 are inserted into the insertion holes 15 in the lower case 10 and are disposed on the mount stage 17. Since the mount stage 17 has the stopper wall 17a formed thereon, the circuit connecting portions 12a can be placed easily and reliably on the mount stage 17 simply by the insertion until it comes into contact with the stopper wall 17a. Then, the fixing belts 3 are provided so as to cover the pair of rubber belts 12. This brings the lower case 10, the rubber belts 12, and the fixing belts 3 together.

Before and after or simultaneously with the operation described above, the positive terminal member 50 and the negative terminal member 60 are fixed to the battery frame 30 by using the positive terminal holding portion 33 and the negative terminal holding portion 34, respectively. This causes the positive terminal member 50 and the negative terminal member 60 to be combined integrally with the battery frame 30 as shown in Fig. 5. The battery side contact portion 52 of the positive terminal member 50 is disposed inside the circumferential wall portion 31 through the first terminal opening portion 36, and the battery side contact portion 63 of the negative terminal member 60 is disposed below the top wall portion 32 through the second terminal opening portion 39.

Next, the battery frame 30 combined with the positive terminal member 50 and the negative terminal member 60 is put inside the guide walls 41 of the holding portion 40 and is placed on the bottom wall portion 10a. As shown in Fig. 4, the battery frame 30 is mounted such that the stage 35 is put into the spacing S formed between the guide walls 41 and the engagement wall 42 engages with the recess portion 37. This can hold the battery frame 30 on the bottom wall portion 10a while defining the orientation in the circumferential direction and defining the position in the diameter direction.

Subsequently, the circuit board 25 having the antenna portion 27 mounted thereon is overlaid on the battery frame 30. The circuit board 25 can be stably overlaid by engaging the engagement recess portion 25b with the engagement wall 42. After the antenna holding portion 28 is overlaid on the circuit board 25, the fixing screw 18 is used to integrally fasten and fix the circuit board 25 and the antenna holding portion 28 to the lower case 10 as shown in Fig. 2.

This causes the circuit connecting portions 12a of the pair of rubber belts 12 to conduct to the circuit board 25 and causes the board side contact portion 54 of the positive terminal member 50 and the board side contact portion 64 of the negative terminal member 60 to conduct to the positive pattern and the negative pattern on the circuit board 25, respectively.

Next, the upper case 11 is combined with the lower case 10. Finally, the button battery 4 is housed into the battery frame 30 through the battery insertion opening 16, and then the battery lid 70 is rotated relatively to the lower case 10 with the lid body 71 of the battery lid 70 inserted in the battery insertion opening 16, thereby engaging the bayonet protrusion 80 with the bayonet engagement groove 73a. Thus, the battery lid 70 can be fixed to the lower case 10 in the bayoneting.

Since the fixing causes the flange portion 72 of the battery lid 70 to press the O-ring 82 and abut on the stepped portion 81, the battery insertion opening 16 is reliably sealed. The button battery 4 is sandwiched and held between the battery lid 70 and the circuit board 25, so that the battery side contact portion 52 of the positive terminal member 50 comes into contact with the outer circumferential face 4b and conducts thereto, and the battery side contact portion 63 of the negative terminal member 60 comes into contact with the negative electrode face 4a and conducts thereto.

With these steps, the assembly of the heart beat measuring apparatus 1 is completed.

In particular, according to the heart beat measuring apparatus 1 of the present embodiment, the battery frame 30 is included independently of the case body 2 formed of the lower case 10 and the upper case 11, the case body 2 and the battery frame 30 can be molded individually unlike the conventional structure including the case body 2 having the integral battery housing portion. Consequently, the battery frame 30 can be easily molded by using a mold dedicated to the battery frame 30, and the battery frame 30 can be formed precisely while suppressing variations in shape after the molding.

In addition, since the moldability can be given a higher priority in the material selection, the molding can be performed more easily and precisely. This allows the formation of the top wall portion 32 of the battery frame 30 serving as the battery stage with a uniform and reduced thickness, and the resulting product can be reduced in thickness.

Since the lower case 10 forming part of the case body 2 can also be provided by using a mold dedicated to the lower case 10, the molding can be facilitated, and variations in shape after the molding can be suppressed. The formation of the battery housing portion in the lower case 10 as conventional is not required, so that the lower case 10 can be simplified in shape.

Since the battery frame 30 can be precisely formed as described above, the button battery 4 can be housed in the battery frame 30 through the battery insertion opening 16 with the battery guide face 31a reliably abutting on the outer circumferential face 4b of the button battery 4 to achieve the stable positioning of the button battery 4 by the reliable control of the position in the diameter direction.

In addition to the stable position of the button battery 4 in the diameter direction, the fixing of the positive terminal member 50 to the battery frame 30 through the positive terminal holding portion 33 can provide the stable and sufficient contact between the button battery 4 and the battery side contact portion 52. The resulting heart beat measuring apparatus 1 can have high quality with high resistance to vibrations, shocks and the like, and improved operation reliability.

Since the battery frame 30 has the top wall portion 32 serving as the battery stage, certain spacing can be provided between the circuit board 25 and the button battery 4. Even if a shock is given during the insertion or drop of the button battery 4, the negative terminal member 60 can be prevented from being crushed or deformed. As a result, the stable and sufficient contact can also be made between the button battery 4 and the battery side contact portion 63 of the negative terminal member 60 to further improve the operation reliability.

If the battery stage is not included and the negative terminal member 60 is soldered to the circuit board 25, a shock given during the insertion or drop of the button battery 4 may apply a stress which would crush and plastically deform the battery side contact portion 63 of leaf spring shape between the circuit board 25 and the button battery 4.

According to the heart beat measuring apparatus 1 of the present embodiment, however, such a problem hardly occurs.

Since the holding portion 40 can be used to fit the battery frame 30 onto the bottom wall portion 10a by defining the orientation in the circumferential direction, the positive terminal member 50 and the negative terminal member 60 can be positioned at the preset positions on the circuit board 25. The board side contact portion 54 of the positive terminal member 50 and the board side contact portion 64 of the negative terminal member 60 can be precisely brought into contact with the positive pattern and the negative pattern on the circuit board 25, respectively, to reliably provide the conduction path between the button battery 4 and the circuit board 25 through the positive terminal member 50 and the negative terminal member 60.

In addition, the positive terminal member 50 and the negative terminal member 60 can be fixed to the battery frame 30 to eliminate the work of soldering them to the circuit board 25, for example, and to easily simplify the assembly process since they can be integrated with the battery frame 30.

Specifically in the conventional configuration in which the positive terminal member 50 and the negative terminal member 60 are soldered to the circuit board 25, the positions of the positive terminal member 50 and the negative terminal member 60 vary in association with errors and the like of the position of the attachment of the circuit board 25, and when the button battery 4 is inserted, it may crush the battery side contact portion 52 of the positive terminal member 50 or may be separate from the battery side contact portion 52 to reduce the contact pressure to cause an unstable contact, for example.

According to the heart beat measuring apparatus 1 of the present embodiment, however, such a problem hardly occurs.

The bayoneting enables the mounting of the battery lid 70 with a single motion and easily reduces the size of the lower case 10 as compared with sliding. In particular, since the lower case 10 can be molded without consideration of the battery frame 30, the bayonet protrusion 80 can be formed precisely. This allows the mounting of the battery lid 70 with reduced rattles, and the button battery 4 can be easily housed inside the battery frame 30 stably.

The technical scope of the present invention is not limited to the embodiment described above, and various modifications can be made without departing from the scope of the present invention as defined by the claims.

For example, although the embodiment has been described by using the heart beat measuring apparatus 1 as an example of the electronic device, the present invention is not limited thereto, and a pedometer or stopwatch may be used, by way of example. Any electronic device that includes the button battery 4 therein may be used.

Although the embodiment described above includes the battery frame 30 of topped cylindrical shape having the circumferential wall portion 31 and the top wall portion 32, the battery frame may have an annular shape which does not have the top wall portion 32, has at least the circumferential wall portion 31, and can be combined with the positive terminal member 50. However, the battery frame 30 preferably has the top wall portion 32 and can be combined with the negative terminal member 60 as in the embodiment.

Although the embodiment described above employs the bayoneting used to fix the battery lid 70, the present invention is not limited thereto, and the sliding may be used. In this case, the lower case 10 can be precisely molded into a shape necessary for fixing through the sliding without consideration of the battery frame 30, so that the battery lid 70 can be mounted with reduced rattles.

However, the bayoneting is preferably used to fix the battery lid 70 as in the embodiment.

## Claims

1. An electronic device (1) including a case body (2) for accommodating a battery (4), comprising:
a battery frame (30) housed in the case body such that the battery frame is mounted on a bottom wall portion (10a) of the case body, the battery being housable in the battery frame;
a holding portion (40) provided on the bottom wall portion and removably holding the battery frame on the bottom wall portion such that a battery insertion opening (16) formed in the bottom wall portion communicates with the interior of the battery frame;
a circuit board (25) housed in the case body, which can conduct to the battery through a positive terminal member (50) and a negative terminal member (60); and
a battery lid (70) removably fixed to the bottom wall portion to cover the battery insertion opening,
wherein the battery frame includes:
a circumferential wall portion (31) for surrounding the battery housable therein in a diameter direction; and
a positive terminal holding portion (33) fixing the positive terminal member such that a battery side contact portion (52) of the positive terminal member is disposed inside the circumferential wall portion (31), and
a portion of an inner circumferential face (31a) of the circumferential wall portion serves as a battery guide face for abutting on an outer circumferential face of the battery to position the battery in the diameter direction and bring the battery into contact with the battery side contact portion of the positive terminal member.

2. The electronic device according to claim 1, wherein the battery frame includes:
a top wall portion (32) connected to the circumferential wall portion (31) and placed between the circuit board and the interior of the battery frame to abut on the battery; and
a negative terminal holding portion (34) fixing the negative terminal member (60) onto the top wall portion such that a battery side contact portion (63) of the negative terminal member can contact the battery.

3. The electronic device according to claim 1 or 2, wherein the holding portion (40) includes an engagement portion (42) engaging with the battery frame (30) and defining an orientation of the battery frame in a circumferential direction through the engagement.

4. The electronic device according to any one of claims 1 to 3, wherein the battery insertion opening (16) is provided with bayonet protrusions (80) protruding inward in a diameter direction and disposed at intervals in a circumferential direction, and
the battery lid (70) is provided with a bayonet hook (73) having a bayonet engagement groove (73a) engaging with the bayonet protrusion when the battery lid is rotated relatively to the case body.

5. The electronic device according to any one of claims 1 to 4, wherein a pair of electrically conductive belts (12) connected to the circuit board (25) and contactable with a living body surface are attached to the case body, and
the circuit board (25) is provided with a detection circuit portion (26) for detecting living body information based on a potential difference produced between the pair of electrically conductive belts.
